# EUROPEAN PATENT SPECIFICATION

(11) **EP 3 408 771 B1**
(45) Date of publication and mention of the grant of the patent: **08.11.2023**
(21) Application number: 17701852.0
(22) Date of filing: 24.01.2017
(51) Int. Cl.: G16H 30/00

(54) **PREDICTIVE MODEL FOR OPTIMIZING CLINICAL WORKFLOW**
PRÄDIKTIVES MODELL ZUR OPTIMIERUNG VON KLINISCHEM ARBEITSFLUSS
MODÈLE PRÉDICTIF POUR OPTIMISATION DE FLUX DE TRAVAIL CLINIQUE

(30) Priority: 27.01.2016 EP 16152878
(43) Date of publication of application: 05.12.2018
(73) Proprietor: Koninklijke Philips N.V., 5656 AG Eindhoven (NL)
(72) Inventor: AMTHOR, Thomas, Erik, 5656 AE Eindhoven (NL); SÉNÉGAS, Julien, 5656 AE Eindhoven (NL); STEHLE, Thomas, Heiko, 5656 AE Eindhoven (NL); HANSIS, Eberhard, Sebastian, 5656 AE Eindhoven (NL)
(74) Representative: Philips Intellectual Property & Standards
(86) International application number: PCT/EP2017/051433
(87) International publication number: WO 2017/129564

(56) References cited:
- WO-A1-2015/099843
- NAVEED EJAZ ET AL: "MRT letter: Visual attention driven framework for hysteroscopy video abstraction", MICROSCOPY RESEARCH AND TECHNIQUE., vol. 76, no. 6, 30 March 2013 (2013-03-30) , pages 559-563, XP055284821, GB ISSN: 1059-910X, DOI: 10.1002/jemt.22205

## Description

### FIELD OF THE INVENTION

The invention relates to a system and a method for optimizing a clinical workflow. The invention further relates to a workstation and imaging apparatus comprising the system. The invention further relates to a computer readable medium comprising instructions for causing a processor system to perform the method. The invention further relates to a computer readable medium comprising the predictive model, and to a use of a predictive model for optimizing a clinical workflow.

### BACKGROUND OF THE INVENTION

In the field of radiology, optimizing the patient and examination workflow, henceforth jointly referred to 'clinical workflow', typically has a large impact on cost and quality of care. Such workflow optimization may be in terms of efficiency as well as diagnostic value of the workflow. Data analytics plays an increasingly important role in hospital management, and may also be used in performing such workflow optimization.

Document MRT Letter: Visual Attention Driven Framework for Hysteroscopy Video Abstraction" by NAVEED EJAZ et al discloses a visual attention inspired salient frame extraction scheme for summarizing hysteroscopy videos. The proposed scheme tries to extract only those frames which provide an unobstructed view of the female reproductive organs. The proposed scheme exploits the strengths of visual attention model combined with domain knowledge for efficient extraction of key frames.

However, one of the main difficulties arising when trying to optimize a clinical workflow is to define suitable performance indicators and to measure and evaluate their value. For example, it may be that certain combinations of Magnetic Resonance Imaging (MRI) sequences, parameters and coils, a certain group of patients, and/or a certain behavior of the medical staff may correlate positively or negatively with the diagnostic value of the medical images acquired during a patient exam. Finding these correlations may involve evaluating many acquired medical images and setting their diagnostic value out against information indicating the conditions under which these medical images have been acquired. This usually represents too much of an effort to be done routinely in clinical practice.

### SUMMARY OF THE INVENTION

It would be advantageous to obtain a system or method which facilitates optimizing a clinical workflow, thereby reducing the effort required for such optimization.

The following aspects of the invention involve generating a predictive model which is predictive of the diagnostic value of medical images acquired by a particular clinical workflow given the workflow metadata of the clinical workflow. The generated predictive model may be used to identify one or more adjustments of the particular clinical workflow so as to improve the diagnostic value of the acquired medical images. To generate the predictive model, the diagnostic value of a medical image is estimated, based on an estimate of the physician's attention during its review, and set out against workflow metadata indicative of the clinical workflow which resulted in the acquisition of the particular medical image.

The invention is defined by the appended claims.

A further aspect of the invention provides a computer readable medium comprising transitory or non-transitory data representing instructions for causing a processor system to perform the method according to any one of the above claims.

A further aspect of the invention provides a computer readable medium comprising transitory or non-transitory data representing a predictive model, the predictive model being predictive of a diagnostic value of medical images acquired by a particular clinical workflow based on workflow metadata of the particular clinical workflow.

A further aspect of the invention provides a use of a predictive model to identify an adjustment of a clinical workflow which, according to the predictive model, results in a higher diagnostic value of the medical images acquired during the clinical workflow.

The above measures involve correlating, using a machine learning technique, an estimated diagnostic value of one or more medical images against workflow metadata indicative of the clinical workflow that establishes the acquisition of the one or more medical images. Here, 'indicative of' refers to the workflow metadata indicating least part of the clinical workflow which is to be carried out, or is carried out, or has been carried out. A non-limiting example is that the workflow metadata is indicative of the used imaging modality, e.g., MRI, the used MRI sequences, the used MRI parameters and coils, etc. Another non-limiting example is that the workflow metadata is indicative of the imaged body region.

Said correlation is performed for different patient exams, and thereby for different clinical workflows, resulting in the input for the machine learning technique being constituted by pairs of i) estimated diagnostic value and ii) workflow metadata. Effectively, the estimated diagnostic values may be considered as an 'answer vector' in the machine learning technique, whereas the workflow metadata may be considered as 'input vector'.

The diagnostic value may be estimated from viewing actions having been taken by the physician using an image viewer. Here, the term 'image viewer' refers to software and/or hardware used to review the medical images, e.g., a software application running on a workstation. Typically, such viewing actions are indicative of the attention the physician is paying to a particular medical image, and may thus represent an 'attention value' of the physician. The viewer log of the image viewer may thus be analyzed to estimate the diagnostic value of the one or more medical images. For that purpose, use is made of an attention metric which embodies a set of assumptions about how different viewing behavior of the physician as represented by the viewing actions correlates with different diagnostic values. For example, the attention metric may map a particular type of viewing action to a higher diagnostic value, while mapping another viewing action to a lower diagnostic value. Here, the diagnostic value may represent a quantification of the value of the medical images for diagnosis, e.g., expressed as a scalar ranging from 0.0, referring to a medical image being unsuitable for medical diagnosis, to 1.0, referring to a medical image being highly suitable for medical diagnosis. The diagnostic value may also be expressed in any other suitable way.

The machine learning technique provides as output a predictive model. The predictive model is constituted by machine-parsable data which allows predicting a diagnostic value of medical images given the workflow metadata of a clinical workflow associated with their acquisition. Effectively, the predictive model may be used as a look-up table, e.g., to 'look-up' the diagnostic value of the medical images using the associated workflow metadata as input, without having necessarily to be structured as a look-up table.

The above measures have the effect that the predictive model allows a particular clinical workflow to be optimized with reduced effort, given that workflow metadata is typically already available in the clinical environment, and the diagnostic value is automatically, or at least semi-automatically estimated. As such, it is not needed for a user, such as a physician, to manually find correlations between workflow parameters and the diagnostic quality of the acquired medical images. Advantageously, the optimization may be performed in routine clinical practice without greatly disturbing said clinical practice.

It is noted that different type of workflow metadata may be used for the machine learning than is subsequently used for the 'look-up' in the diagnostic model.

The obtaining of the workflow metadata may comprise obtaining a system log of a medical apparatus or medical system used in carrying out the clinical workflow. System logs have been found to contain relevant information which correlates well with the diagnostic value of the one or more medical images acquired during the clinical workflow. However, a subsequent 'look-up' using the generated predictive model may be performed using different types of workflow metadata. For example, the workflow metadata may have been generated for a hypothetical clinical workflow rather than having been gathered, e.g., in the form of a system log, during an actual clinical workflow. This enables the clinical workflow still to be modified before being actually carried out.

The system log may be of an imaging apparatus used in the acquisition of the one or more medical images. The system log of the imaging apparatus has been found to be highly predictive of the diagnostic value of the resulting medical images. An example of an imaging apparatus is a MRI scanner or a Computer Tomography (CT) scanner.

Optionally, the attention metric maps the one or more viewing actions to the diagnostic value on the basis of at least one of: an occurrence or number of occurrences of a particular viewing action, a temporal order of the one or more viewing actions, a viewing duration of a particular medical image, and a viewing frequency of the particular medical image. The occurrence or number of occurrences of a particular viewing action may be indicative of, or directly represent an attention value which in turn relates to a particular diagnostic value. For example, the occurrence of a delineation of a region in a medical image may indicate a particular interest of the physician in the medical image, and thereby indicate a relatively high diagnostic value. Likewise, multiple diverging brightness and/or contrast adjustments may indicate that the acquired medical image does not provide a good view of a particular region of interest and thus that its diagnostic value is sub-optimal. Another example is that a long viewing duration, or multiple repeated views, of a medical image may indicate a physician's heightened interest in the medical image.

Optionally, the one or more viewing actions comprise at least one of: a zooming action, a contrast adjustment, a brightness adjustment, a switching to another medical image, a deletion of a medical image, and a delineation of an anatomical structure in the medical image. Such viewing actions are deemed to be indicative of the attention of the physician, and thereby of the diagnostic value of the one or more medical images.

Optionally, the method further comprises querying the physician to obtain user input on the diagnostic value, wherein the estimating of the diagnostic value is further based on the user input. In addition to estimating the diagnostic value based on the viewing actions, the physician may also be directly queried, e.g., using a dialog box in the image viewer's graphical user interface, on the diagnostic value. The physician's input may then be used to supplement the estimation based on the viewing actions, or possibly replace the estimated diagnostic value. It is noted that the estimation, rather than direct querying, of the diagnostic value may remain relevant, e.g., when the physician refrains from providing user input, or to refine coarse user input provided by the physician.

Optionally, the method further comprises accessing a radiology report associated with the patient exam, wherein the estimating of the diagnostic value is further based on an analysis of the radiology report. The radiology report may be indicative of the diagnostic value of the one or more medical images as the radiology report typically reports on the clinical relevance of said medical images. For example, if the radiology report does not include a diagnosis and/or clinical findings, it may indicate a lesser or no diagnostic value of the medical images. By taking into account the radiology report in addition to the viewing actions, the diagnostic value can be more reliably estimated.

Optionally, the method further comprises analyzing the radiology report using a natural language processing technique. By using a natural language processing technique, as known per se in the art, the radiology report as generated by the physician may be directly analyzed, e.g., without having it to be generated in a machine-parsable format.

Optionally, the method further comprises analyzing the predictive model to identify an adjustment of the particular clinical workflow which, according to the predictive model, results in a higher diagnostic value of the medical images. Having generated a predictive model, the predictive model may be used to optimize clinical workflows. For example, the predictive model may be used to predict the diagnostic value of medical images acquired by several variations of a clinical workflow. For that purpose, workflow metadata may be generated being indicative of the several variations of the clinical workflow. The variation yielding the best diagnostic value according to the predictive model may then be selected to be actually carried out, selected as default clinical workflow, etc. Various other users of the predictive model are equally conceivable. For example, a visualization may be generated based on the predictive model which may enable the physician or other user him/herself to identify an optimization using the visualization.

It will be appreciated by those skilled in the art that two or more of the above-mentioned embodiments, implementations, and/or optional aspects of the invention may be combined in any way deemed useful.

Modifications and variations of the system, the imaging apparatus, the workstation, and/or any of the claimed computer readable media, which correspond to the described modifications and variations of the method, can be carried out by a person skilled in the art on the basis of the present description.

A person skilled in the art will appreciate that the method may be applied to multi-dimensional image data, e.g., to two-dimensional (2D), three-dimensional (3D) or four-dimensional (4D) images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 shows an embodiment of a system for generating a predictive model, with the system comprising an optional user interface subsystem configured for visualizing output to a radiologist and for receiving user input from the radiologist;
Fig. 2 shows another embodiment of a system for generating a predictive model, communicating with an imaging apparatus, PACS server and image viewer;
Fig. 3 shows a visualization of information derived from a system log of an imaging apparatus, namely a number of scan repetitions per body part;
Fig. 4 shows another visualization of information derived from a system log of an imaging apparatus, namely an exam timeline;
Fig. 5 shows a user interface of an image viewer used by a radiologist to review a medical image, with the user interface comprising a user feedback dialog box;
Fig. 6 shows a method for generating a predictive model; and
Fig. 7 shows a computer readable medium comprising instructions for causing a processor system to perform the method.

It should be noted that the figures are purely diagrammatic and not drawn to scale. In the figures, elements which correspond to elements already described may have the same reference numerals.

### List of reference numbers

The following list of reference numbers is provided for facilitating the interpretation of the drawings and shall not be construed as limiting the claims.
020 metadata repository
022 workflow metadata
040 report repository
042 data representing radiology report
060 display
062 display data
080 user input device
082 user input data
100, 102 system for generating predictive model
120 metadata input interface
140 report data input interface
160 processor
180 user interface subsystem
182 display output
184 user device input
200 imaging apparatus
202 system log of imaging apparatus
204 medical image(s)
210 PACS server
212 medical image metadata
220 image viewer
222 viewer log of image viewer
224 user feedback data
300 visualization of system log - bar chart of scan repetitions
302 number axis
310 visualization of system log - exam timeline
312 time axis
400 user interface of image viewer
410 medical image
412 signal dropout region
420 icons representing viewing actions
430 user feedback dialog box
500 method for generating predictive model
510 obtaining workflow metadata
520 obtaining attention value
530 estimating diagnostic value
540 applying machine learning technique
570 computer readable medium
580 non-transitory data

### DETAILED DESCRIPTION OF EMBODIMENTS

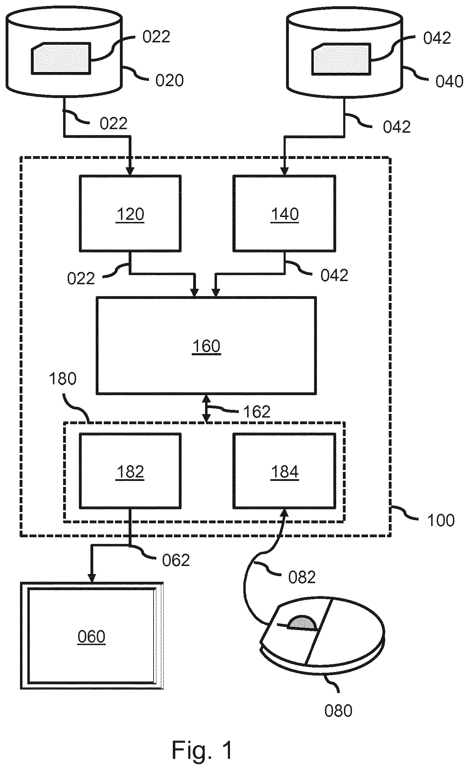

Fig. 1 shows a first embodiment of a system for generating a predictive model. The predictive model may be used for optimizing a clinical workflow, with the clinical workflow resulting in acquisition of one or more medical images during a patient exam.

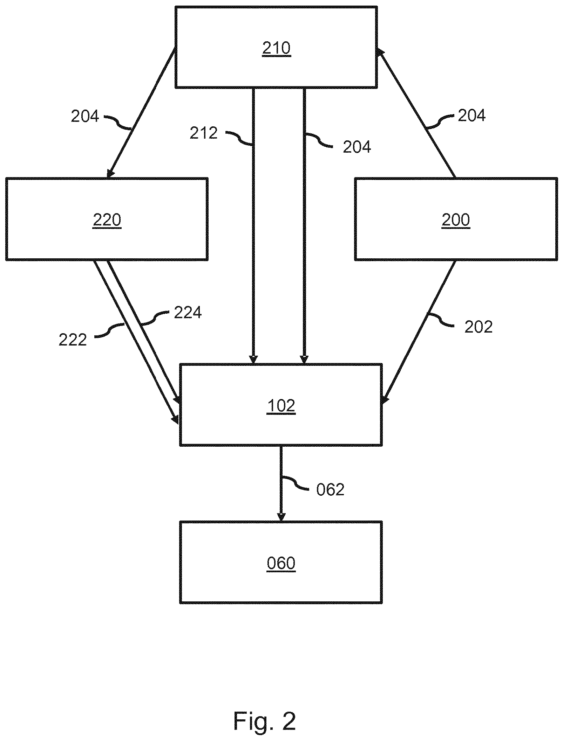

The system 100 is shown to comprise a metadata input interface 120 for accessing workflow metadata 022 which is indicative of the clinical workflow. As will be explained further with reference to Figs. 2-4, such workflow metadata 022 may take various forms and may be obtained from various sources. In the example of Fig. 1, the metadata input interface 120 is shown to be connected to an externally located metadata repository 020 which comprises the workflow metadata 022. Alternatively, the workflow metadata 022 may be accessed from an internal data storage of the system 100. In general, the metadata input interface 120 may take various forms, such as a network interface to a local or wide area network, e.g., the Internet, a storage interface to an internal or external data storage, etc. An example of a local area network is that within hospital or other clinical site.

The processor 160 is configured for, during operation of the system 100, determining an attention value which characterizes a review of the one or more medical images by a physician, and for estimating a diagnostic value of the one or more medical images to the physician for reaching a clinical diagnosis. In this respect, it is noted that henceforth reference will be made to the physician being, by way of example, a radiologist. The described operations are performed by the processor for different clinical workflows, e.g., involving different patient exams, thereby obtaining a plurality of diagnostic values. The metadata input interface 120 is configured for obtaining the respective plurality of workflow metadata, e.g., each being indicative of a respective clinical workflow. The processor 160 is further configured for, during operation of the system 100, applying a machine learning technique to the plurality of diagnostic values and the plurality of workflow metadata to generate a predictive model, the predictive model being predictive of the diagnostic value of medical images acquired by a particular clinical workflow based on the workflow metadata of the particular clinical workflow.

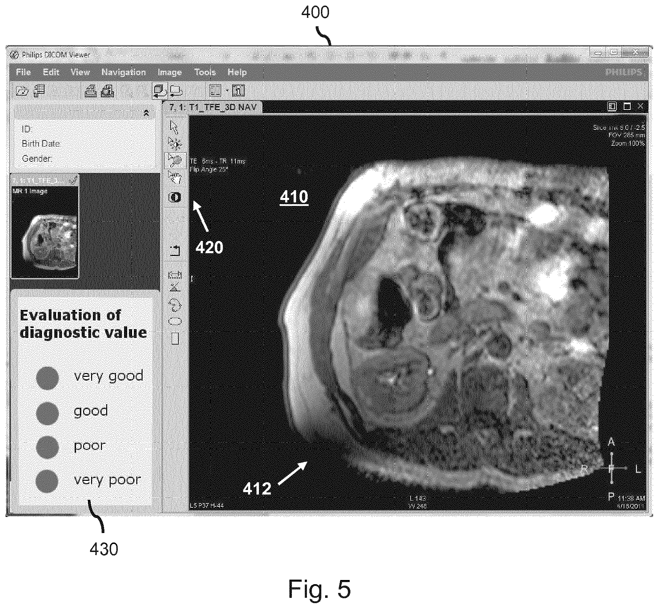

Examples of workflow metadata will be further discussed with reference to Figs. 2-4, whereas the determining of the attention value and the estimating of the diagnostic value using the attention value will be further discussed with reference to Fig. 5. In particular, as attention value, a viewer log of an image viewer may be obtained which is indicative of one or more viewing actions performed by the radiologist using the image viewer. Unless otherwise noted, the attention value may be directly represented by the viewing actions. As such, any reference to determining the attention value may be understood as determining the viewing actions from the viewer log of the image viewer.

In general, however, the predictive model may be generated as follows. The workflow metadata may be used to form a multi-dimensional feature vector. This vector may be associated with a respective diagnostic value, as estimated by the system. A non-limiting example is that the diagnostic value may be a real number between 0 and 1. The machine learning technique may be used to segment the feature vector's feature space into regions in which the feature vectors are associated with a same or similar diagnostic value. Machine learning techniques which may be used to generate the predictive model include, but are not limited to, Support Vector Machines (SVM), decision trees/forests, neural networks / deep learning, k-Nearest Neighbors (kNN), etc. The predictive model may be generated to be indicative of these regions as well as the diagnostic value within each region.

Having generated the predictive model, the predictive model may be used to predict the most probable diagnostic value of a particular clinical workflow, namely by determining in which region the feature vector of the particular workflow metadata falls. The predictive model may also be used to identify an adjustment of the particular clinical workflow which improves the diagnostic value of the acquired medical images. For example, a further feature vector may be identified which is associated with a high diagnostic value. The differences between both feature vectors may represent the adjustment(s) to be made. By selecting a further feature vector which is similar to the feature vector of the particular workflow metadata, the number and/or amount of adjustment(s) can be minimized. Similarity between feature vectors may be quantified in a manner known per se, e.g., based on a distance measure. An adjustment may then be recommended to the radiologist or other user. Examples of workflow adjustments include, but are not limited to, utilization of a better suited MR sequence, a temporal reordering of MR sequences, utilization of specific MR coils, improved patient communication (talk to him/her so the patient is more relaxed), etc.

With further reference to Fig. 1, the system 100 is further shown to comprise a report data input interface 140 which is connected to a report repository 040. As such, the system 100 of Fig. 1 is enabled to access one or more radiology reports 042. This optional aspect of the system 100 will be further explained with reference to Fig. 5.

The system 100 of Fig. 1 is further shown to comprise an user interface subsystem 180 comprising a display output 182 for generating display data 062 for display on a display 060, and a user device input 184 for receiving user input data 082 provided by a user input device 080 operable by the user. The user input device 080 may take various forms, including but not limited to a computer mouse 080, touch screen, keyboard, etc. The user input interface 180 may be of a type which corresponds to the type of user input device 080, i.e., it may be a thereto corresponding user device interface. Together, the display output 182 and the user device input 184 enable a user to interact with the system 100, based on data communication 162 between the processor 160 and the user interface subsystem 180. For example, system 100 may display the predictive model, or a visual representation of the predictive model, on the display 060. Additionally or alternatively, the system 100 may visualize one or more adjustments to the clinical workflow which have been identified using the predictive model. The user interface subsystem 180 may also be used to query a radiologist on the diagnostic value, as will be further explained with reference to Fig. 5.

In general, the system 100 may be embodied as, or in, a single device or apparatus, such as a workstation or imaging apparatus. The workstation may be co-configured as image viewer, e.g., by being configured for running a software application which enables a radiologist to review one or more medical images. The device or apparatus may comprise one or more microprocessors which execute appropriate software. The software may have been downloaded and/or stored in a corresponding memory, e.g., a volatile memory such as RAM or a non-volatile memory such as Flash. Alternatively, the units of the system may be implemented in the device or apparatus in the form of programmable logic, e.g., as a Field-Programmable Gate Array (FPGA). In general, each unit of the system may be implemented in the form of a circuit. It is noted that the system 100 may also be implemented in a distributed manner, e.g., involving different devices or apparatuses. For example, the distribution may be in accordance with a client-server model.

Fig. 2 shows a second embodiment of a system 102 for generating a predictive model. Here, various options are shown of the system 102 obtaining workflow metadata which is indicative of a particular clinical workflow. A first example is that the system 102 may receive a system log 202 from an imaging apparatus 200, e.g., via a network such as that of a Hospital Information System (HIS). The system log 202 may comprise workflow information and information about which medical image(s) 204 have been acquired by the imaging apparatus 200 during the particular clinical workflow. A second example is that a Picture Archiving and Communication System (PACS) server 210 may provide medical image metadata 212 of the acquired medical image(s) 204 to the system 102. Medical image metadata 212 has been found to be indicative of the clinical workflow. The medical image metadata 212 may be accompanied by the one or more medical images 204 themselves. Further shown in Fig. 2 is an image viewer 220 for enabling the radiologist to review the medical images 204. The image viewer 220 is shown to receive the medical images 204 from the PACS server 210 and to provide a viewer log 222 and user feedback data 224 to the system, e.g., via a network. Both types of data may be used to (better) estimate the diagnostic value of the medical images 204, and will be further explained with reference to Fig. 5. It is noted that an example of an image viewer 220 is a radiologist's workstation.

The system 102 may use all, or a selection of the obtained (meta)data as input in the machine learning technique to correlate the estimated diagnostic value of the one or more medical images 204 with the information known about the clinical workflow, as well as other information such as patient information. This may enable clinical workflow parameters, including those of the patient exam, which correlate with a good/bad diagnostic value, to be identified and then visualized or otherwise used to improve the clinical workflow.

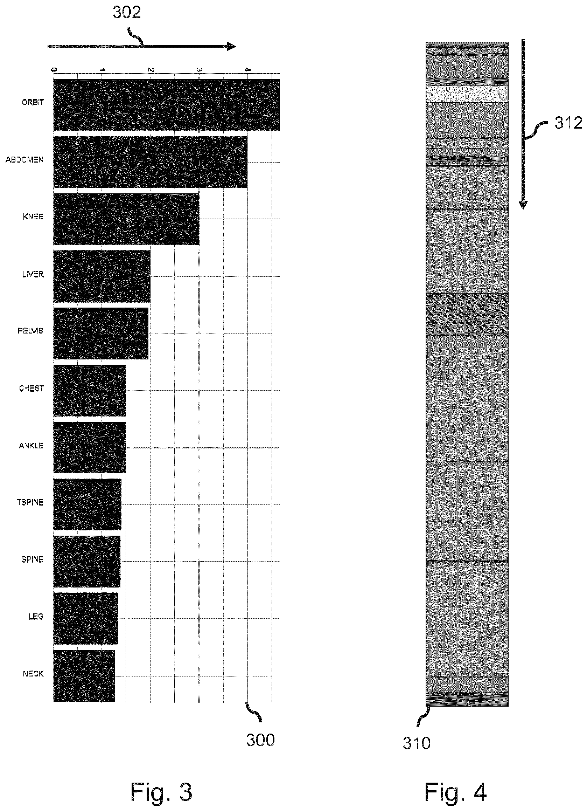

Fig. 3 shows a visualization of information derived from a system log of an imaging apparatus, namely a bar chart 300 representing a number of scan repetitions per body part. In particular, Fig. 3 shows along a horizontal axis 302 the average number of scan repetitions per exam for different examined body parts for one particular imaging apparatus, e.g., a MRI scanner, while setting out along the vertical axis the different examined body parts. It will be appreciated that the number of scan repetitions is indicative of the clinical workflow, and may correlate with the diagnostic value of the acquired images. For example, the fact that the abdomen is scanned repeatedly may relate to image quality issues caused by the patients moving or not holding their breath appropriately while the images were acquired. By correlating this information with the estimated diagnostic value of the images, the generated predictive model may indicate, for example, which patients are especially excited and tend to move a lot. It may also indicate that, for example, scans are repeated unnecessarily, so that the workflow could be improved by not repeating the scan in some cases.

Fig. 4 shows another visualization of information derived from a system log of an imaging apparatus, namely an exam timeline 310. In particular, Fig. 4 shows an exam timeline 310 reconstructed from log file information of an imaging apparatus, e.g., as obtained from a system log. Here, the vertical axis 312 corresponds to the time axis. Different intensities and patterns denote different events. Examples of events indicated by such log file information may be the occurrence of patient table motion, a diagnostic scan, a survey scan, and/or an automatic reference scan taking place. In the example of Fig. 4, the hatching indicates that a scan has been aborted (here, the aborted scan was repeated after a 30-second break). The region of lightest intensity represents idle time. Although the precise events occurring in the exam timeline 310 are not of particular relevance, it will be appreciated that these events, possibly including their order, duration, etc., are indicative of the clinical workflow, and may correlate with the diagnostic value of the acquired images. For example, the fact that a particular scan was aborted may relate to the patient being nervous and moving a lot. Such an event may therefore be indicative of image qualities below average also for some other scans of this exam. As another example, a patient table motion following an aborted scan may indicate that the patient had not been positioned correctly at the beginning of the exam and that, consequently, all images taken before the repositioning may be of limited diagnostic value.

Fig. 5 shows a user interface 400 of an image viewer used by a radiologist to review a medical image 410. Such image viewing functionality may be provided by the system generating the predictive model itself, e.g., by the system comprising a user interface subsystem as shown in Fig. 1, or by the system being integrated into an image viewer, e.g., a radiologist's workstation. Alternatively, the image viewer may be separately provided from the system, but may be modified to provide the following additional functionality.

Namely, in addition to the radiologist being enabled to review one or more medical images 410 using the user interface 400 and to select various viewing actions, represented by icons 420 in the user interface 400, the user interface 400 may establish a user feedback dialog box 430 on-screen which enables the radiologist to actively provide feedback on the diagnostic value of the currently displayed medical image 310. For example, the radiologist may have the option to select between 'very good', 'good', 'poor' and 'very poor' using on-screen buttons. In this particular example, the radiologist may select 'poor' in view of the medical image 410 comprising a signal dropout 412. Such user feedback may be used by the system to replace or augment the estimation of the diagnostic value based on the attention value. An example of the latter is that user feedback may be used where available, whereas otherwise the diagnostic value may be estimated by the system. Another example is that the diagnostic value as indicated by the radiologist may be refined by, or used to refine, the estimation of the diagnostic value based on the attention value. It is noted that instead of querying the radiologist for the diagnostic value, the radiologist may also be queried for an image quality. The image quality may reveal problems during the image acquisition, and may be a good indicator for diagnostic quality and thus the diagnostic value of the acquired medical images. A bad image quality, for example caused by noise, signal dropout, or image reconstruction artifacts, will in many cases also lead to a bad diagnostic value of the image. However, a good image quality does not always imply a good diagnostic value, because the image may simply not cover the region of interest, or the image contrast was chosen in such a way that is not suited to answer the clinical question.

The image viewer may, if needed, be further modified to make available a viewer log which is indicative of one or more viewing actions performed by the radiologist using the image viewer. Accordingly, the attention value may be determined based on an analysis of the viewer log. For that purpose, the image viewer may measure certain information, including but not limited to the viewing time and viewing frequency of each medical image. Furthermore, viewing actions selected by the radiologist may be logged, including but not limited to zooming, adjustments in image contrast and brightness, frequent alternations (switching back/forth) between certain medical images, the deletion of a medical image, manual delineation of anatomical structures in a medical image, etc.

The attention value may be determined from the logged viewing actions. Alternatively, the logged viewing actions may be already considered as representing attention values. For example, a long viewing duration may denote a high attention value, whereas a deletion of a medical image may denote a low attention value. Accordingly, the diagnostic value may be directly estimated based on the information in the viewer log. For example, an attention metric may be used which embodies a set of assumptions about the radiologist's viewing behavior and which correlates the occurrence, number of occurrences, temporal order, etc., of the viewing actions with the diagnostic value. A specific example may be that frequent adjustments of brightness and contrast may indicate that the image contrast was not optimal and thus that the diagnostic value is relatively low. Another specific example is that medical images viewed for only for a very short time or medical images deleted by the radiologist may indicate to the system that their diagnostic value is relatively low and that the corresponding scans may be omitted in an optimized version of the clinical workflow.

In general, there may exist a radiology report which is associated with the patient exam. The diagnostic value may be estimated based on an analysis of the radiology report, for example using natural language processing (NLP) or similar tools in order to extract information about the diagnostic value of the images from the textual description.

It will be appreciated that the invention as claimed may also be applied to a review of medical images by a clinician other than a radiologist. As such, where appropriate, the term 'radiologist' may be replaced by 'clinician.

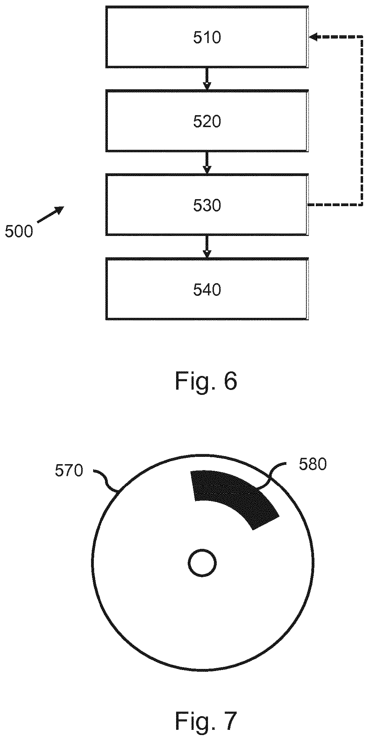

Fig. 6 shows a method 500 for generating a predictive model, which may correspond to an operation of the system as described with reference to Figs. 1-5.

The method 500 comprises, in an operation titled "OBTAINING WORKFLOW METADATA", obtaining 510 workflow metadata indicative of the clinical workflow. The method 500 further comprises, in an operation titled "OBTAINING ATTENTION VALUE", obtaining 520 an attention value, the attention value at least in part characterizing a review of the one or more medical images by a radiologist. The method 500 further comprises, in an operation titled "ESTIMATING DIAGNOSTIC VALUE", estimating 530 a diagnostic value of the one or more medical images to the radiologist for reaching a clinical diagnosis, wherein said estimating is performed based on the attention value and an attention metric, the attention metric mapping different attention values to different diagnostic values. The above steps may be performed for different clinical workflows to obtain a plurality of diagnostic values and a respective plurality of workflow metadata, as indicated in Fig. 6 by a dashed arrow indicating the steps being repeated. The method 500 further comprises, in an operation titled "APPLYING MACHINE LEARNING TECHNIQUE", applying 540 a machine learning technique to the plurality of diagnostic values and the plurality of workflow metadata to generate a predictive model, the predictive model being predictive of the diagnostic value of medical images acquired by a particular clinical workflow based on the workflow metadata of the particular clinical workflow.

It will be appreciated that the above operation may be performed in any suitable order, e.g., consecutively, simultaneously, or a combination thereof, subject to, where applicable, a particular order being necessitated, e.g., by input/output relations. For example, the workflow metadata may be obtained before or after the estimation of the diagnostic value, the plurality of diagnostic values may be estimated in parallel, etc.

The method 500 may be implemented on a computer as a computer implemented method, as dedicated hardware, or as a combination of both. As also illustrated in Fig. 7, instructions for the computer, e.g., executable code, may be stored on a computer readable medium 570, e.g., in the form of a series 580 of machine readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 7 shows an optical disc 570.

Alternatively, the computer readable medium of Fig. 7 may comprise transitory or non-transitory data representing a predictive model as generated by the system and method, with the data being stored in a transitory or non-transitory manner, e.g., in the form of a series of machine readable physical marks and/or as a series of elements having different electrical, e.g., magnetic, or optical properties or values.

It will be appreciated that, in accordance with the abstract of the present specification, a system and method are provided for generating a predictive model for use in optimizing a clinical workflow. The predictive model may be generated as follows. Workflow metadata is obtained which is indicative of the clinical workflow. An attention value is obtained which characterizes a radiologist's review of one or more medical images acquired during the clinical workflow. A diagnostic value of the one or more medical images is then estimated based on the attention value. The above steps are performed for different clinical workflows. A machine learning technique is then applied to the resulting plurality of diagnostic values and the plurality of workflow metadata to generate the predictive model. The generated predictive model is predictive of the diagnostic value of medical images acquired by a particular clinical workflow given the workflow metadata of the particular clinical workflow. Advantageously, the predictive model may be used to modify the clinical workflow so as to increase the diagnostic value of the acquired images.

Examples, embodiments or optional features, whether indicated as non-limiting or not, are not to be understood as limiting the invention as claimed.

It will be appreciated that the invention also applies to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware.

## Claims

1. A computer-implemented method (500) for optimizing a clinical workflow, the clinical workflow resulting in acquisition of one or more medical images (204, 310) during a patient exam, the method comprising the steps of:
- obtaining (510) workflow metadata (022, 202, 212) indicative of the clinical workflow, wherein the obtaining the workflow metadata comprises obtaining a system log of a medical apparatus or medical system used in carrying out the clinical workflow;
- obtaining (520) a viewer log of an image viewer used by a physician to review the one or more medical images, the viewer log being indicative of one or more viewing actions performed by the physician using the image viewer;
- estimating (530) a diagnostic value of the one or more medical images to the physician for reaching a clinical diagnosis, wherein said estimating comprises mapping the one or more viewing actions to the diagnostic value using an attention metric, wherein the attention metric embodies a set of assumptions about how different viewing behavior of the physician as represented by the viewing actions correlates with different diagnostic values;
wherein the above steps are performed for different clinical workflows to obtain a plurality of diagnostic values and a respective plurality of workflow metadata, the method further comprising:
- applying (540) a machine learning technique to the plurality of diagnostic values and the plurality of workflow metadata to generate a predictive model, the predictive model being predictive of the diagnostic value of medical images acquired by a particular clinical workflow based on the workflow metadata of the particular clinical workflow; and
- optimizing the particular clinical workflow by predicting the diagnostic value of medical images acquired by several variations of the clinical workflow and selecting the variation which yields the best diagnostic value as an optimized variant of the clinical workflow.

2. The computer-implemented method (500) according to claim 1, wherein the system log (202) is of an imaging apparatus (200) used in the acquisition of the one or more medical images (204).

3. The computer-implemented method (500) according to claim 1, wherein the attention metric maps the one or more viewing actions to the diagnostic value on the basis of at least one of: an occurrence or number of occurrences of a particular viewing action, a temporal order of the one or more viewing actions, a viewing duration of a particular medical image, and a viewing frequency of the particular medical image.

4. The computer-implemented method (500) according to any one of claim 1 to 3, wherein the one or more viewing actions comprise at least one of: a zooming action, a contrast adjustment, a brightness adjustment, a switching to another medical image, a deletion of a medical image, and a delineation of an anatomical structure in the medical image.

5. The computer-implemented method (500) according to any one of the above claims, further comprising querying the physician to obtain user input (224) on the diagnostic value, and wherein the estimating of the diagnostic value is further based on the user input.

6. The method (500) according to any one of the above claims, further comprising accessing a radiology report (042) associated with the patient exam, and analyzing the radiology report using a natural language processing technique to extract information indicative of the diagnostic value of the one or more medical images from a textual description in the radiology report, wherein the diagnostic value is estimated further based on said extracted information.

7. A computer readable medium (570) comprising transitory or non-transitory data (580) representing instructions for causing a processor system to perform the method according to any one of the above claims.

8. A use of a predictive model as defined by claim 1 to optimize a clinical workflow by using the predictive model to predict the diagnostic value of medical images acquired by several variations of the clinical workflow and selecting the variation which yields the best diagnostic value as an optimized variant of the clinical workflow.

9. A system (100, 102) for generating a predictive model for optimizing a clinical workflow, the clinical workflow resulting in acquisition of one or more medical images (204, 310) during a patient exam, the system comprising:
- an input interface (120) configured for obtaining workflow metadata (022, 202, 212) indicative of the clinical workflow, wherein the obtaining the workflow metadata comprises obtaining a system log of a medical apparatus or medical system used in carrying out the clinical workflow;
- a memory comprising instruction data representing a set of instructions;
- a processor (160) configured to communicate with the input interface and the memory and to execute the set of instructions, wherein the set of instructions, when executed by the processor, cause the processor to:
i) obtain a viewer log of an image viewer used by a physician to review the one or more medical images, the viewer log being indicative of one or more viewing actions performed by the physician using the image viewer; and
ii) estimate a diagnostic value of the one or more medical images to the physician for reaching a clinical diagnosis, wherein said estimating comprises mapping the one or more viewing actions to the diagnostic value using an attention metric, wherein the attention metric embodies a set of assumptions about how different viewing behavior of the physician as represented by the viewing actions correlates with different diagnostic values;
wherein the set of instructions, when executed by the processor, cause the processor to estimate a plurality of diagnostic values for different clinical workflows and the input interface (120) is configured for obtaining a respective plurality of workflow metadata, wherein the set of instructions, when executed by the processor, cause the processor to:
- apply a machine learning technique to the plurality of diagnostic values and the plurality of workflow metadata to generate a predictive model, the predictive model being predictive of the diagnostic value of medical images acquired by a particular clinical workflow based on the workflow metadata of the particular clinical workflow.

10. A workstation or imaging apparatus (200) comprising the system (100, 102) according to claim 9.

## Patentansprüche

1. Computerimplementiertes Verfahren (500) zur Optimierung eines klinischen Arbeitsablaufs, wobei der klinische Arbeitsablauf zur Erfassung eines oder mehrerer medizinischer Bilder (204, 310) während einer Patientenuntersuchung führt, wobei das Verfahren die folgenden Schritte umfasst:
- Erhalten (510) von Arbeitsablauf-Metadaten (022, 202, 212), die den klinischen Arbeitsablauf angeben, wobei das Erhalten der Arbeitsablauf-Metadaten das Erhalten eines Systemprotokolls eines medizinischen Geräts oder medizinischen Systems umfasst, das bei der Durchführung des klinischen Arbeitsablaufs verwendet wird;
- Erhalten (520) eines Betrachterprotokolls eines Bildbetrachters, den ein Arzt zur Betrachtung des einen oder der mehreren medizinischen Bilder verwendet, wobei das Betrachterprotokoll eine oder mehrere Betrachtungsaktionen anzeigt, die der Arzt unter Verwendung des Bildbetrachters durchgeführt hat;
- Schätzen (530) eines diagnostischen Werts des einen oder der mehreren medizinischen Bilder für den Arzt, um eine klinische Diagnose zu erhalten, wobei das Schätzen das Zuordnen der einen oder mehreren Betrachtungsaktionen zum diagnostischen Wert unter Verwendung einer Aufmerksamkeitsmetrik umfasst, wobei die Aufmerksamkeitsmetrik Folgendes verkörpert:
Reihe von Annahmen darüber, wie unterschiedliches Sehverhalten des Arztes, dargestellt durch die Sehhandlungen, mit unterschiedlichen diagnostischen Werten korreliert;
wobei die oben genannten Schritte für unterschiedliche klinische Arbeitsabläufe durchgeführt werden, um mehrere Diagnosewerte und jeweils mehrere Arbeitsablauf-Metadaten zu erhalten, wobei das Verfahren weiterhin Folgendes umfasst:
- Anwenden (540) einer Technik des maschinellen Lernens auf die mehreren diagnostischen Werte und die mehreren Arbeitsablauf-Metadaten, um ein Vorhersagemodell zu erzeugen, wobei das Vorhersagemodell den diagnostischen Wert medizinischer Bilder vorhersagt, die durch einen bestimmten klinischen Arbeitsablauf auf der Grundlage der Arbeitsablauf-Metadaten erfasst wurden des jeweiligen klinischen Arbeitsablaufs; und
- Optimierung des jeweiligen klinischen Arbeitsablaufs durch Vorhersage des diagnostischen Werts medizinischer Bilder, die mit mehreren Varianten des klinischen Arbeitsablaufs erfasst wurden, und Auswahl der Variante, die als optimierte Variante des klinischen Arbeitsablaufs den besten diagnostischen Wert liefert.

2. Computerimplementiertes Verfahren (500) nach Anspruch 1, wobei das Systemprotokoll (202) von einem Bildgebungsgerät (200) stammt, dass bei der Erfassung des einen oder der mehreren medizinischen Bilder (204) verwendet wird.

3. Computerimplementiertes Verfahren (500) nach Anspruch 1, wobei die Aufmerksamkeitsmetrik die eine oder mehreren Betrachtungshandlungen dem Diagnosewert auf der Grundlage von mindestens einem von Folgendem zuordnet: einem Auftreten oder einer Anzahl von Erscheinungen einer bestimmten Betrachtungshandlung, eine zeitliche Reihenfolge der einen oder mehreren Betrachtungsaktionen, eine Betrachtungsdauer eines bestimmten medizinischen Bildes und eine Betrachtungshäufigkeit des bestimmten medizinischen Bildes.

4. Computerimplementiertes Verfahren (500) nach einem der Ansprüche 1 bis 3, wobei die eine oder mehreren Betrachtungsaktionen mindestens eine von Folgendem umfassen: eine Zoomaktion, eine Kontrastanpassung, eine Helligkeitsanpassung, ein Wechsel zu einem anderen medizinischen Gerät Bild, eine Löschung eines medizinischen Bildes und eine Abgrenzung einer anatomischen Struktur im medizinischen Bild.

5. Computerimplementierte Verfahren (500) nach einem der oben genannten Ansprüche, dass ferner das Befragen des Arztes umfasst, um eine Benutzereingabe (224) zum Diagnosewert zu erhalten, und wobei die Schätzung des Diagnosewerts weiterhin auf dem Benutzer basiert Eingang.

6. Verfahren (500) nach einem der oben genannten Ansprüche, das außerdem den Zugriff auf einen mit der Patientenuntersuchung verbundenen Radiologiebericht (042) und die Analyse des Radiologieberichts unter Verwendung einer Verarbeitungstechnik natürlicher Sprache umfasst, um Informationen zu extrahieren, die den diagnostischen Wert angeben des einen oder der mehreren medizinischen Bilder aus einer Textbeschreibung im radiologischen Bericht, wobei der diagnostische Wert basierend auf den extrahierten Informationen weiter geschätzt wird.

7. Computerlesbares Medium (570), das vorübergehende oder nichtflüchtige Daten (580) umfasst, die Anweisungen darstellen, um ein Prozessorsystem zu veranlassen, das Verfahren gemäß einem der oben genannten Ansprüche auszuführen.

8. Verwendung eines Vorhersagemodells nach Anspruch 1 zur Optimierung eines klinischen Arbeitsablaufs durch Verwendung des Vorhersagemodells zur Vorhersage des diagnostischen Werts medizinischer Bilder, die durch mehrere Variationen des klinischen Arbeitsablaufs erfasst wurden, und Auswahl der Variation, die den besten diagnostischen Wert liefert eine optimierte Variante des klinischen Arbeitsablaufs.

9. System (100, 102) zum Erzeugen eines Vorhersagemodells zur Optimierung eines klinischen Arbeitsablaufs, wobei der klinische Arbeitsablauf zur Erfassung eines oder mehrerer medizinischer Bilder (204, 310) während einer Patientenuntersuchung führt, wobei das System umfasst:
- eine Eingabeschnittstelle (120), die zum Erhalten von Arbeitsablauf-Metadaten (022, 202, 212) konfiguriert ist, die den klinischen Arbeitsablauf angeben, wobei das Erhalten der Arbeitsablauf-Metadaten das Erhalten eines Systemprotokolls eines medizinischen Geräts oder medizinischen Systems umfasst, das bei der Durchführung des klinischen Arbeitsablaufs verwendet wird;
- einen Speicher, der Befehlsdaten umfasst, die einen Befehlssatz darstellen;
- einen Prozessor (160), der so konfiguriert ist, dass er mit der Eingabeschnittstelle und dem Speicher kommuniziert und den Befehlssatz ausführt, wobei der Befehlssatz, wenn er vom Prozessor ausgeführt wird, den Prozessor dazu veranlasst:
i) ein Betrachterprotokoll eines Bildbetrachters zu erhalten, der von einem Arzt zur Betrachtung des einen oder der mehreren medizinischen Bilder verwendet wird, wobei das Betrachterprotokoll eine oder mehrere Betrachtungsaktionen anzeigt, die der Arzt unter Verwendung des Bildbetrachters durchgeführt hat; Und
ii) Schätzen eines diagnostischen Werts des einen oder der mehreren medizinischen Bilder für den Arzt, um eine klinische Diagnose zu erhalten, wobei das Schätzen das Zuordnen der einen oder mehreren Betrachtungsaktionen zum diagnostischen Wert unter Verwendung einer Aufmerksamkeitsmetrik umfasst, wobei die Aufmerksamkeitsmetrik einen Satz von verkörpert Annahmen darüber, wie unterschiedliches Sehverhalten des Arztes, dargestellt durch die Sehhandlungen, mit unterschiedlichen diagnostischen Werten korreliert;
wobei der Befehlssatz bei Ausführung durch den Prozessor dazu führt, dass der Prozessor mehrere Diagnosewerte für unterschiedliche klinische Arbeitsabläufe schätzt, und die Eingabeschnittstelle (120) zum Erhalten einer jeweiligen Vielzahl von Arbeitsablaufmetadaten konfiguriert ist, wobei der Befehlssatz, bei Ausführung durch den Prozessor den Prozessor dazu veranlassen:
- Anwenden einer Technik des maschinellen Lernens auf die Vielzahl von Diagnosewerten und die Vielzahl von Workflow-Metadaten, um ein Vorhersagemodell zu generieren, wobei das Vorhersagemodell den diagnostischen Wert medizinischer Bilder vorhersagt, die durch einen bestimmten klinischen Workflow auf der Grundlage der Workflow-Metadaten des jeweiligen Patienten erfasst wurden klinischer Arbeitsablauf.

10. Workstation oder Bildgebungsvorrichtung (200), die das System (100, 102) nach Anspruch 9 umfasst.

## Revendications

1. Procédé mis en oeuvre par ordinateur (500) pour optimiser un flux de travail clinique, le flux de travail clinique aboutissant à l'acquisition d'une ou plusieurs images médicales (204, 310) pendant un examen d'un patient, le procédé comprenant les étapes suivantes:
- obtenir (510) des métadonnées de flux de travail (022, 202, 212) indicatives du flux de travail clinique, l'obtention des métadonnées de flux de travail comprenant l'obtention d'un journal système d'un appareil médical ou d'un système médical utilisé dans la réalisation du flux de travail clinique;
- obtenir (520) un journal de visualisation d'une visionneuse d'images utilisée par un médecin pour examiner la ou les images médicales, le journal de visualisation étant indicatif d'une ou plusieurs actions de visualisation effectuées par le médecin à l'aide de la visionneuse d'images;
- estimer (530) une valeur diagnostique de la ou des images médicales pour le médecin afin d'atteindre un diagnostic clinique, ladite estimation comprenant la mise en correspondance de la ou des actions de visualisation avec la valeur diagnostique à l'aide d'une métrique d'attention, la métrique d'attention incarnant un ensemble d'hypothèses sur la manière dont différents comportements de visualisation du médecin, représentés par les actions de visualisation, sont en corrélation avec différentes valeurs diagnostiques; dans lequel les étapes ci-dessus sont effectuées pour différents flux de travail cliniques afin d'obtenir une pluralité de valeurs diagnostiques et une pluralité respective de métadonnées de flux de travail, le procédé comprenant en outre:
- appliquer (540) une technique d'apprentissage automatique à la pluralité de valeurs de diagnostic et à la pluralité de métadonnées de flux de travail pour générer un modèle prédictif, le modèle prédictif étant prédictif de la valeur diagnostique d'images médicales acquises par un flux de travail clinique particulier sur la base des métadonnées de flux de travail du flux de travail clinique particulier; et
- optimiser le flux de travail clinique particulier en prédisant la valeur diagnostique d'images médicales acquises par plusieurs variantes du flux de travail clinique et en sélectionnant la variation qui donne la meilleure valeur diagnostique en tant que variante optimisée du flux de travail clinique.

2. Procédé mis en oeuvre par ordinateur (500) selon la revendication 1, dans lequel le journal système (202) est celui d'un appareil d'imagerie (200) utilisé dans l'acquisition de la ou des images médicales (204).

3. Procédé mis en oeuvre par ordinateur (500) selon la revendication 1, dans lequel la métrique d'attention mappe la ou les actions de visualisation à la valeur de diagnostic sur la base d'au moins l'un parmi: une occurrence ou un nombre d'occurrences d'une action de visualisation particulière, un ordre temporel de la ou des actions de visualisation, une durée de visualisation d'une image médicale particulière et une fréquence de visualisation de l'image médicale particulière.

4. Procédé mis en oeuvre par ordinateur (500) selon l'une quelconque des revendications 1 à 3, dans lequel la ou les actions de visualisation comprennent au moins l'un parmi: une action de zoom, un réglage du contraste, un réglage de la luminosité, un passage à une autre image médicale, une suppression d'une image médicale, et une délimitation d'une structure anatomique dans l'image médicale.

5. Procédé mis en oeuvre par ordinateur (500) selon l'une quelconque des revendications ci-dessus, comprenant en outre l'interrogation du médecin pour obtenir une entrée de l'utilisateur (224) sur la valeur diagnostique, et dans lequel l'estimation de la valeur diagnostique est en outre basée sur l'entrée utilisateur.

6. Procédé (500) selon l'une quelconque des revendications ci-dessus, comprenant en outre l'accès à un rapport de radiologie (042) associé à l'examen du patient, et l'analyse du rapport de radiologie à l'aide d'une technique de traitement du langage naturel pour extraire des informations indicatives de la valeur diagnostique de la ou des images médicales à partir d'une description textuelle dans le rapport de radiologie, la valeur diagnostique étant en outre estimée sur la base desdites informations extraites.

7. Support lisible par ordinateur (570) comprenant des données transitoires ou non transitoires (580) représentant des instructions pour amener un système processeur à exécuter le procédé selon l'une quelconque des revendications ci-dessus.

8. Utilisation d'un modèle prédictif tel que défini par la revendication 1 pour optimiser un flux de travail clinique en utilisant le modèle prédictif pour prédire la valeur diagnostique d'images médicales acquises par plusieurs variantes du flux de travail clinique et en sélectionnant la variation qui donne la meilleure valeur diagnostique comme une variante optimisée du flux de travail clinique.

9. Système (100, 102) pour générer un modèle prédictif pour optimiser un flux de travail clinique, le flux de travail clinique aboutissant à l'acquisition d'une ou plusieurs images médicales (204, 310) lors d'un examen d'un patient, le système comprenant:
- une interface d'entrée (120) configurée pour obtenir des métadonnées de flux de travail (022, 202, 212) indicatives du flux de travail clinique, l'obtention des métadonnées de flux de travail comprenant l'obtention d'un journal système d'un appareil médical ou d'un système médical utilisé dans la réalisation du flux de travail clinique;
- une mémoire comprenant des données d'instructions représentant un ensemble d'instructions;
- un processeur (160) configuré pour communiquer avec l'interface d'entrée et la mémoire et pour exécuter l'ensemble d'instructions, l'ensemble d'instructions, lorsqu'il est exécuté par le processeur, amenant le processeur à:
i) obtenir un journal de visualisation d'une visionneuse d'images utilisée par un médecin pour examiner la ou les images médicales, le journal de visualisation étant indicatif d'une ou plusieurs actions de visualisation effectuées par le médecin à l'aide de la visionneuse d'images; et
ii) estimer une valeur diagnostique de la ou des images médicales pour le médecin afin d'atteindre un diagnostic clinique, ladite estimation comprenant le mappage de la ou des actions de visualisation avec la valeur diagnostique à l'aide d'une métrique d'attention, la métrique d'attention représentant un ensemble des hypothèses sur la manière dont différents comportements de visualisation du médecin, représentés par les actions de visualisation, sont en corrélation avec différentes valeurs diagnostiques; dans lequel l'ensemble d'instructions, lorsqu'il est exécuté par le processeur, amène le processeur à estimer une pluralité de valeurs de diagnostic pour différents flux de travail cliniques et l'interface d'entrée (120) est configurée pour obtenir une pluralité respective de métadonnées de flux de travail, dans lequel l'ensemble d'instructions, lorsqu'il est exécuté par le processeur, amène le processeur à:
- appliquer une technique d'apprentissage automatique à la pluralité de valeurs diagnostiques et à la pluralité de métadonnées de flux de travail pour générer un modèle prédictif, le modèle prédictif étant prédictif de la valeur diagnostique d'images médicales acquises par un flux de travail clinique particulier sur la base des métadonnées de flux de travail du flux de travail clinique particulier.

10. Poste de travail ou appareil d'imagerie (200) comprenant le système (100, 102) selon la revendication 9.
